# EUROPEAN PATENT APPLICATION

(11) **EP 3 730 164 A1**
(43) Date of publication of application: **28.10.2020**
(21) Application number: 18893291.7
(22) Date of filing: 17.08.2018
(51) Int. Cl.: A61L 31/14, A61L 31/16

(54) **DRUG-LOADED IMPLANTED MEDICAL DEVICE AND PREPARATION METHOD THEREFOR**

(30) Priority: 22.12.2017 CN 201711405582
(71) Applicant: Shanghai MicroPort Medical (Group) Co., Ltd., Shanghai 201203 (CN)
(72) Inventor: CHEN, Lu, Shanghai 201203 (CN); HU, Yan, Shanghai 201203 (CN); LI, Junfei, Shanghai 201203 (CN); WANG, Xuan, Shanghai 201203 (CN); HU, Qifeng, Shanghai 201203 (CN)
(74) Representative: Patentship Patentanwaltsgesellschaft mbH
(86) International application number: PCT/CN2018/100916
(87) International publication number: WO 2019/119834

(57) **Abstract**

A drug-loaded implanted medical device and a preparation method therefor. The drug-loaded implanted medical device comprises a device body and grooves (2) distributed on the surface of the device body, and at least one of the grooves (2) is loaded with a solid drug (11); the solid drug (11) is a crystal or has a form in which a crystal coexists with an amorphous body. The preparation method is implemented by loading the solid drug (11) into at least one of the grooves (2) by means of solution crystallization.

## Description

### TECHNICAL FIELD

The present application relates to the field of medical devices and, in particular, to a drug-loaded implantable medical device and a preparation method thereof.

### BACKGROUND

Cardiovascular disease is one of the most health-threatening diseases. Since the commercialization of Cypher, the world's first drug-eluting stent (DES) developed by the US company, Johnson & Johnson, was approved by the US FDA in 2003, various such DES's have been developed and vastly used. The success of DES's is praised as another milestone in the domain of interventional cardiology, following bare-metal stents, and opens a new era for this field. Large-scale randomized, double-blind clinical studies have shown that DES's can significantly reduce the incidence of intra-stent restenosis (RS) and major cardiac adverse events (MACE).

Most existing DES's adopt a drug-loading design in which a drug-containing coating is applied to the surface of a bare stent. The coating that bonds the drug to the stent allows controlled release of the drug and is also called a drug carrier. However, since the drug is distributed across the entire stent surface in this design, it is unable to achieve the effective control of the drug release in one or more desired directions, leading to part of the drug not to be absorbed by the vessel wall. The drug carriers usually implemented as polymer matrixes, which tend to cause inflammatory response in vessels. In addition, the drug coating applied to stent portions that may experience significant changes in shape tends to fall off during the delivery or expansion of the stent. Such fallen off particles will flow with the blood, which tends to form a blood clot may threaten human health. CN 101879102A published a drug loaded groove carrying-type drug eluting stent, comprising a stent body and a drug coating, drug-loading grooves formed on an external surface of the stent body. The drug coating is loaded into grooves, and the drug coating comprises a biological degradable polymer matrix and an active drug. The polymer matrix allows the drug to be controlled-released.

The above drug-eluting stent enables bonding of the coating to the stent while allowing release of the drug towards a target blood vessel wall, thus providing an improved effectiveness and safety during the use of the drug stent. However, it still requires using a polymer to carry the drug. Although this polymer is degradable, after the stent is implanted into human body, due to the long-term existence or degradation of the polymer on the surface of the stent, it is possible to cause continuous inflammatory response, which in turn causes a risk of a delayed vascular endothelialization or a late vessel restenosis.

### SUMMARY

The present application provides a drug-loaded implantable medical device and a preparation method thereof to solve the problem that existing implantable medical devices must rely on the drug carrier to load the drug on the surface of the medical device and perform the controlled-release of drug, which brings clinical risks.

To solve the above problem, the present application provides a drug-loaded implantable medical device, which comprises a body and grooves distributed on a surface of the stent, at least one of the grooves having a solid drug loaded therein, the solid drug being a crystal or having a form in which a crystal and an amorphous body coexist.

Optionally, the solid drug is one or more selected from the group consisting of paclitaxel, sirolimus and sirolimus derivatives.

Optionally, the sirolimus derivative is everolimus or zotarolimus.

Optionally, the grooves each have a depth of from 5µm to 100 µm, preferably from 15µm to 75 µm.

Optionally, the grooves each have an opening area of from 200µm² to 75,000 µm², preferably from 2,000µm² to15,000 µm², more preferably from 4,000µm² to 8,000 µm².

Optionally, the opening of the groove is rectangular, elliptic or circular. The grooves may be continuously or discontinuously distributed. The grooves may be distributed in a regular or random manner. The grooves may be distributed across the entire surface of the stent body or some portions thereof. The grooves may be distributed with a uniform density or a region-dependent density.

Optionally, the body is made of one or more materials selected from the group consisting of metal, ceramic, carbon and macromolecular polymer. Preferably, the body is made of one or more materials selected from the group consisting of cobalt-based alloy, stainless steel, titanium alloy, activated ceramic, carbon and polylactic acid.

The present application also provide s a preparation method of the drug-loaded implantable medical device as defined above, in which the solid drug is loaded in the at least one of the grooves using a solution crystallization process.

Optionally, the solution crystallization process is performed by obtaining a drug solution through dissolution of an active drug in a mixed solvent, disposing the drug solution into the at least one of the grooves, and after volatilization or evaporation of the mixed solvent, obtaining an implantable medical device in which the solid drug is entirely or partially crystallized in the at least one of the grooves.

Optionally, the mixed solvents may comprise a solvent A and a solvent B, the solvent A selected from acetone, isopropanol, methanol or ethanol, preferably selected from isopropanol or ethanol, the solvent B selected from n-heptane, acetonitrile, n-propyl acetate or ethyl acetate, preferably selected from n-heptane or n-propyl acetate.

Optionally, a volume ratio of the solvent A to solvent B is from 2:1 to 1:99.

Optionally, the active drug is present in the drug solution at a mass percentage of from 0.1% to 20%, preferably from 2% to 10%.

Optionally, the drug solution is subjected to two or more processes of spraying and solvent volatilization or evaporation, so as to be loaded in the groove in batches.

Optionally, the spraying is conducted at an ambient temperature of from 20°C to 40°C, preferably from 25°C to 30°C.

Optionally, the drug solution is sprayed for 2-25 times per groove, each spray providing an amount of from 300 pL to 600 pL of the drug solution.

The technical solution of present application eliminates clinical risks brought by the common use of polymer coatings to load drugs in prior art, and achieves the design of a polymer-free drug-loaded implantable medical device such as the drug stent. Such design is able to provide controlled-release of drugs with a significantly improved safety as well as achieve a drug release performance comparable to that of conventional drug-loaded implantable medical devices having a polymer coating.

### BRIEF DESCRIPTION OF THE DRAWINGS

Fig. 1 is a structural schematic of a stent of the prior art.
Fig. 2 schematically illustrates a drug-loading groove of the stent surface of Fig. 1 and a solid drug loaded therein.
Fig. 3 is an electron micrograph of the drug-loaded stent of Embodiment 1.
Fig. 4 is an electron micrograph of a solid drug in a drug-loading groove of Embodiment 1.

### DETAILED DESCRIPTION

For ease of understanding, the provided drug-loaded implantable medical device will be described below combining with specific embodiments. It is to be understood that these embodiments are provided merely for illustration of present application rather than limiting the scope thereof in any sense.

For ease of explanation, the technical solution of present application implemented using merely one specific stent is described below and reference can be made for implementations of other types of drug-loaded implantable medical devices.

The medical device involved in the present application may be either an implantable device or a device for extracorporeal use. It can be either used temporarily in a short term or permanently implanted for a long term. In some embodiment, suitable devices are those typically used to provide medical therapy and/or diagnostics for heart rhythm disorders, heart failures, valve diseases, vascular diseases, diabetes, neurological diseases and disorders, orthopedics, neurosurgery, oncology, ophthalmology, and ENT surgeries. The medical devices involved in present application may include, but are not limited to, stent, stent graft, anastomotic connector, synthetic patch, lead, electrode, needle, guide wire, catheter, sensor, surgical instruments, angioplasty balloon, wound drainage tube, shunt, pipe, infusion sleeve, intraurethral cannula, pellet, implant, blood oxygenator, pump, vascular graft, vascular access port, heart valve, annuloplasty ring, suture, surgical clip, surgical nail, pacemaker, implantable defibrillator, neurostimulator, orthopedic devices, cerebrospinal fluid shunt, implantable drug pump, spinal cage, artificial intervertebral disc, replacement devices for nucleus pulposus, ear canal, intraocular lens and any tubing used in an interventional surgery.

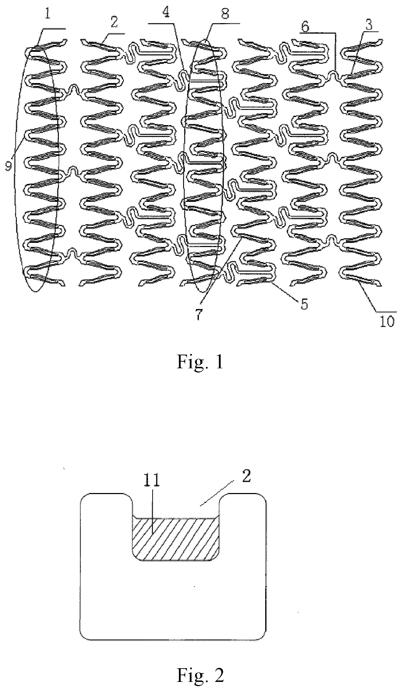

As shown in Fig. 1, the stent is made of cobalt-chromium alloy and constructed from a plurality of main support rings 1, 8 and connecting rods 4, 6 connecting the rings together. Each main support ring consists of a plurality of wavy elements. Each wavy element consists of arc-shaped reinforcing sections 9, straight sections 10 composed of struts and intermediate sections (e.g., 3, 5, 7) connecting the reinforcing sections 9 and straight sections 10. The external surface of each straight section is provided with a groove 2 that can load a drug. In the wavy elements of the main support rings, each straight section 10 has a width of 96 µm, and each reinforcing section 9 has a width of 91 µm. In addition, the intermediate section smoothly links the straight section 10 with the reinforcing section 9, and the stent body has a thickness of 100 µm.

The laser cutting technique is used to form a groove 2 with a substantially rectangular opening. The groove 2 has a depth of from 5µm to 100 µm, preferably from 15µm to75 µm, such as 30 µm, and the groove opening has an area of from 200µm² to 75,000 µm², preferably from 2,000µm² to 15,000 µm², more preferably from 4,000µm² to 8,000 µm². The groove 2 has been processed for use. It is to be noted that the openings of the grooves 2 may be square, elliptic, circular or irregular. Distributions of the grooves 2 on the stent body may be regular or random, may be continuous or discontinuous, may be across the entire surface of the stent body or just present in some portions, may have a uniform density or a region-dependent density.

In general, a solid drug may be loaded in the grooves 2 using the following approach.

A drug solution obtained by dissolving the active drug in mixed solvent is filtered and accurately sprayed into the grooves of the stent body. After volatilization or evaporation of the mixed solvent and drying, a drug stent in which the solid drug is entirely or partially crystallized in each groove is obtained.

Fig. 2 shows that a solid drug 11 is loaded in a groove 2 formed on the surface of a metal stent strut.

Optionally, the drug solution obtained by dissolving the active drug in the mixed solvents may be subjected to two or more processes of spraying and solvent volatilization or evaporation, so as to be disposed in grooves in batches. In this way, a drug-loaded stent with different drug-loading amounts in different grooves or a drug-loaded stent satisfying a specific desired drug release profile can be obtained.

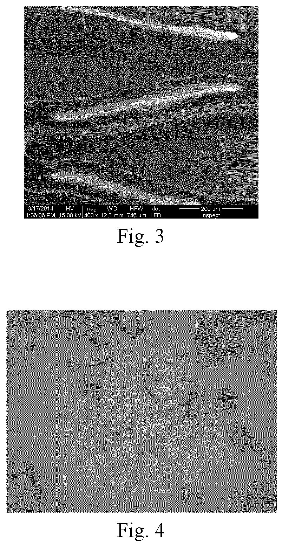

The setup and crystallization will be described in greater detail in following embodiments.
Embodiment 1
   A drug solution containing 5% by weight of sirolimus was obtained by placing sirolimus in 10ml of a methanol/n-propyl acetate (v/v=1:70) mixture at 40°C for 1h and then filtering the solution. The obtained drug solution is accurately sprayed into grooves 2 of a stent body at a controlled amount of 500 pL per groove 2. The stent body was then placed at 20°Cfor natural volatilization or evaporation of the solvents. After natural volatilization or evaporation of the solvents, the spray process was repeated until each groove 2 had been treated for a total of 5 times. After that, irradiating sterilization was performed to the stent body, so as to result in a carrier-free drug stent. As shown in the electron micrographs of Figs. 3 and 4, the crystalline solid drug was present in the carrier-free drug stent.
Embodiment 2
   A drug solution containing 1% by weight of sirolimus was obtained by placing sirolimus in 10ml of an ethanol/n-propyl acetate (v/v=1:2) mixture at 40°C for 1h and then filtering the solution. The obtained drug solution is accurately sprayed into grooves 2 of a stent body at a controlled amount of 500 pL per groove 2. The stent body was then placed at 20°Cfor natural volatilization or evaporation of the solvents. After natural volatilization or evaporation of the solvents, the spray process was repeated until each groove 2 had been treated for a total of 25 times. After that, irradiating sterilization was performed to the stent, so as to result in a carrier-free drug stent.
Embodiment 3
   A drug solution containing 1% by weight of sirolimus was obtained by placing sirolimus in 10ml of an acetone / n-heptane (v/v=1:5) mixture at 35°C for 1h and then filtering the solution. The obtained drug solution is accurately sprayed into grooves 2 of a stent body at a controlled amount of 500 pL per groove 2. The stent body was then placed at 25°Cfor natural volatilization or evaporation of the solvents. After natural volatilization or evaporation of the solvents, the spray process was repeated until each groove 2 had been treated for a total of 25 times. After that, irradiating sterilization was performed to the stent, so as to result in a carrier-free drug stent.
Embodiment 4
   A drug solution containing 1% by weight of sirolimus was obtained by placing sirolimus in 10ml of an isopropanol/n-heptane (v/v=1:1) mixture at 35°C for 1h and then filtering the solution. The obtained drug solution is accurately sprayed into grooves 2 of a stent body at a controlled amount of 500 pL per groove 2. The stent body was then placed at 25°C for natural volatilization or evaporation of the solvents. After natural volatilization or evaporation of the solvents, the spray process was repeated until each groove 2 had been treated for a total of 25 times. After that, irradiating sterilization was performed to the stent, so as to result in a carrier-free drug stent.
Embodiment 5
   A drug solution containing 1% by weight of paclitaxel was obtained by placing paclitaxel in 10ml of a acetone/ethyl acetate (v/v=1:2) mixture at 35°C for 2h and then filtering the solution. The obtained drug solution is accurately sprayed into grooves 2 of a stent body at a controlled amount of 600 pL per groove 2. The stent body was then placed at 25°C for natural volatilization or evaporation of the solvents. After natural volatilization or evaporation of the solvents, the spray process was repeated until each groove 2 had been treated for a total of 2 times. After that, irradiating sterilization was performed to the stent, so as to result in a carrier-free drug stent.
Embodiment 6
   A drug solution containing 6% by weight of paclitaxel was obtained by placing paclitaxel in 10ml of a acetone/acetonitrile (v/v=2:1) mixture at 35°C for 2h and then filtering the solution. The obtained drug solution is accurately sprayed into grooves 2 of a stent body at a controlled amount of 300 pL per groove 2. The stent body was then placed at 20°C for natural volatilization or evaporation of the solvents. After natural volatilization or evaporation of the solvents, the spray process was repeated until each groove 2 had been treated for a total of 2 times. After that, irradiating sterilization was performed to the stent, so as to result in a carrier-free drug stent.
Embodiment 7
   A drug solution containing 3% by weight of paclitaxel was obtained by placing paclitaxel in 10ml of an isopropanol/n-propyl acetate (v/v=1:80) mixture at 35°C for 2h and then filtering the solution. The obtained drug solution is accurately sprayed into grooves 2 of a stent body at a controlled amount of 300 pL per groove 2. The stent body was then placed at 20°C for natural volatilization or evaporation of the solvents. After natural volatilization or evaporation of the solvents, the spray process was repeated until each groove 2 had been treated for a total of 2 times. After that, irradiating sterilization was performed to the stent, so as to result in a carrier-free drug stent.
Embodiment 8
   A drug solution containing 10% by weight of paclitaxel was obtained by placing zotarolimus in 10ml of an isopropanol/n-propyl acetate (v/v=1:1) mixture at 30°C for 0.5h and then filtering the solution. The obtained drug solution is accurately sprayed into grooves 2 of a stent body at a controlled amount of 300 pL per groove 2. The stent body was then placed at 20°C for natural volatilization or evaporation of the solvents. After natural volatilization or evaporation of the solvents, the spray process was repeated until each groove 2 had been treated for a total of 9 times. After that, irradiating sterilization was performed to the stent, so as to result in a carrier-free drug stent.
Embodiment 9
   A drug solution containing 10% by weight of paclitaxel was obtained by placing zotarolimus in 10ml of an isopropanol/n-propyl acetate (v/v=2:1) mixture at 30°C for 0.5h and then filtering the solution. The obtained drug solution is accurately sprayed into grooves 2 of a stent body at a controlled amount of 300 pL per groove 2. The stent body was then placed at 20°C for natural volatilization or evaporation of the solvents. After natural volatilization or evaporation of the solvents, the spray process was repeated until each groove 2 had been treated for a total of 9 times. After that, irradiating sterilization was performed to the stent, so as to result in a carrier-free drug stent.
Embodiment 10
   A drug solution containing 10% by weight of paclitaxel was obtained by placing zotarolimus in 10ml of an isopropanol/n-propyl acetate (v/v=2:1) mixture at 20°C for 0.5h and then filtering the solution. The obtained drug solution is accurately sprayed into grooves 2 of a stent body at a controlled amount of 300 pL per groove 2. The stent body was then placed at 20°C for natural volatilization or evaporation of the solvents. After natural volatilization or evaporation of the solvents, the spray process was repeated until each groove 2 had been treated for a total of 9 times. After that, irradiating sterilization was performed to the stent, so as to result in a carrier-free drug stent.
Embodiment 11
   A drug solution containing 20% by weight of paclitaxel was obtained by placing zotarolimus in 10ml of an isopropanol/n-propyl acetate (v/v=1:10) mixture at 20°C for 0.5h and then filtering the solution. The obtained drug solution is accurately sprayed into grooves 2 of a stent body at a controlled amount of 350 pL per groove 2. The stent body was then placed at 20°C for natural volatilization or evaporation of the solvents. After natural volatilization or evaporation of the solvents, the spray process was repeated until each groove 2 had been treated for a total of 4 times. After that, irradiating sterilization was performed to the stent, so as to result in a carrier-free drug stent.
Embodiment 12
   A drug solution containing 20% by weight of paclitaxel was obtained by placing zotarolimus in 10ml of an isopropanol/n-propyl acetate (v/v=1:50) mixture at 25°C for 0.5h and then filtering the solution. The obtained drug solution is accurately sprayed into grooves 2 of a stent body at a controlled amount of 350 pL per groove 2. The stent body was then placed at 20°C for natural volatilization or evaporation of the solvents. After natural volatilization or evaporation of the solvents, the spray process was repeated until each groove 2 had been treated for a total of 4 times. After that, irradiating sterilization was performed to the stent, so as to result in a carrier-free drug stent.
Embodiment 13
   A drug solution containing 10% by weight of paclitaxel was obtained by placing zotarolimus in 10ml of an isopropanol/n-propyl acetate (v/v=1:99) mixture at 30°C for 0.5h and then filtering the solution. The obtained drug solution is accurately sprayed into grooves 2 of a stent body at a controlled amount of 400 pL per groove 2. The stent body was then placed at 20°C for natural volatilization or evaporation of the solvents. After natural volatilization or evaporation of the solvents, the spray process was repeated until each groove 2 had been treated for a total of 7 times. After that, irradiating sterilization was performed to the stent, so as to result in a carrier-free drug stent.

### Comparative Experiments

Comparative Example: a stent with a polymer coating is prepared in accordance with the method disclosed in CN 101879102 A.

Drug release experiments *in vitro* are performed for comparison between the carrier-free drug-loaded stents prepared in accordance with the above Embodiments and the drug-loaded stent of Comparative Example.

*In vitro* release was performed:
using the small cup method provided in the Chinese Pharmacopoeia with a 5% aqueous solution of sodium dodecyl sulfate (SDS) as a release medium at a temperature of 37°C and a speed of 100 rpm. The results of samples taken respectively at 1h, 8h, 24h, 3d, 7d, 14d are shown in Table 1.

**Table 1**

| Time | | 1h | 8h | 24h | 3d | 7d | 14d |
|---|---|---|---|---|---|---|---|
| Control Group | Comparative Example | 19.6% | 38.5% | 45.5% | 62.4% | 79.2% | 93.9% |
| Experimen tal Group | Embodiment 1 | 25.5% | 45.1% | 53.8% | 70.7% | 85.5% | 97.1% |
| | Embodiment 2 | 22.2% | 40.4% | 47.3% | 66.0% | 81.9% | 94.0% |
| | Embodiment 3 | 23.4% | 40.9% | 48.1% | 63.4% | 75.6% | 90.1% |
| | Embodiment 4 | 18.8% | 39.8% | 48.0% | 65.1% | 82.1% | 98.9% |
| | Embodiment 5 | 13.9% | 31.9% | 40.5% | 57.3% | 74.1% | 95.5% |
| | Embodiment 6 | 15.5% | 33.4% | 41.6% | 55.2% | 76.6% | 96.7% |
| | Embodiment 7 | 16.1% | 34.3% | 40.7% | 55.9% | 72.9% | 96.1% |
| | Embodiment 8 | 21.4% | 35.7% | 44.0% | 58.7% | 83.6% | 98.8% |
| | Embodiment 9 | 22.8% | 42.4% | 49.7% | 65.1% | 84.6% | 99.2% |
| | Embodiment 10 | 19.4% | 39.0% | 46.8% | 61.1% | 80.6% | 95.3% |
| | Embodiment 11 | 12.8% | 31.1% | 38.9% | 52.9% | 67.9% | 86.1% |
| | Embodiment 12 | 20.2% | 40.1% | 47.9% | 64.0% | 79.9% | 95.5% |
| | Embodiment 13 | 18.6% | 36.0% | 45.1% | 65.0% | 83.1% | 99.1% |

As can be seen from the results in Table 1, the technical solution of present application can completely replace conventional drug carriers with crystallization to achieve drug loading and sustained release. Moreover, compared to the solution of using drug carriers, present application is able to fully solve the problem of clinical inflammatory response caused by the drug carrier and thus eliminate the consequent risks of the delayed vascular endothelialization and late vessel restenosis.

It is to be noted that the above embodiments are presented merely for the purpose of illustrating the present application and are not intended to limit it in any sense. Although the present application has been described in detail with reference to the foregoing embodiments, those of ordinary skilled in the art will appreciate that modifications can be made for the technical solutions provided in above embodiments or equivalent replacements can be made for part or all of the technical features. Such modifications or equivalent replacements do not depart the essence of the technical solutions from the scope of the various embodiments of the application.

## Claims

1. A drug-loaded implantable medical device, comprising a body and grooves distributed on a surface of the body, at least one of the grooves having a solid drug loaded therein, the solid drug being a crystal or having a form in which a crystal and an amorphous body coexist.

2. The drug-loaded implantable medical device according to claim 1, wherein the solid drug is one or more selected from the group consisting of paclitaxel, sirolimus and sirolimus derivatives.

3. The drug-loaded implantable medical device according to claim 1, wherein the grooves each have a depth of from 5µm to100 µm, preferably from 15µm to 75 µm.

4. The drug-loaded implantable medical device according to claim 1, wherein the grooves each have an opening area of from 200µm² to 75,000 µm², preferably from 2,000µm² to15,000 µm², more preferably from 4,000µm² to 8,000 µm².

5. A preparation method of a drug-loaded implantable medical device according to any one of claims 1 to 4, wherein the solid drug is loaded in the at least one of the grooves using a solution crystallization process.

6. The method according to claim 5, wherein the solution crystallization process is performed by obtaining a drug solution through dissolution of an active drug in a mixed solvent, disposing the drug solution into the at least one of the grooves, and after volatilization or evaporation of the mixed solvent, obtaining an implantable medical device in which the solid drug is entirely or partially crystallized in the at least one of the grooves.

7. The method according to claim 6, wherein the mixed solvent comprises a solvent A and a solvent B, the solvent A selected from acetone, isopropanol, methanol or ethanol, preferably selected from isopropanol or ethanol, the solvent B selected from n-heptane, acetonitrile, n-propyl acetate or ethyl acetate, preferably selected from n-heptane or n-propyl acetate.

8. The method according to claim 7, wherein a volume ratio of the solvent A to solvent B is from 2:1 to 1:99.

9. The method according to claim 6, wherein the active drug is present in the drug solution at a mass percentage of from 0.1% to 20%, preferably from 2% to 10%.

10. The method according to claim 6, wherein the drug solution is subjected to two or more processes of spraying and solvent volatilization or evaporation, so as to be loaded in the grooves in batches.

11. The method according to claim 10, wherein the spraying is conducted at an ambient temperature of from 20°C to 40°C, preferably from 25°C to 30°C.

12. The method according to claim 10, wherein the drug solution is sprayed for 2-25 times per groove, each spray providing an amount of from 300 pL to 600 pL of the drug solution.
